# EUROPEAN PATENT SPECIFICATION

(11) **EP 3 057 553 B1**
(45) Date of publication and mention of the grant of the patent: **03.01.2018**
(21) Application number: 14784059.9
(22) Date of filing: 13.10.2014
(51) Int. Cl.: A61F 13/511

(54) **SANITARY NAPKIN WITH OPTIMIZED ANIONIC STRIP**
HYGIENEBINDE MIT OPTIMIERTEM ANIONISCHEN STREIFEN
SERVIETTE HYGIÉNIQUE PRÉSENTANT UNE BANDE ANIONIQUE OPTIMISÉE

(30) Priority: 15.10.2013 IT MI20131706
(43) Date of publication of application: 24.08.2016
(73) Proprietor: HNP S.r.l, 64021 Giulianova (TE) (IT)
(72) Inventor: GRILLI, Michele, 64021 Giulianova (TE) (IT)
(74) Representative: Mittler, Andrea
(86) International application number: PCT/EP2014/071921
(87) International publication number: WO 2015/055595

(56) References cited:
- EP-A1- 1 066 825

## Description

The present invention relates to a sanitary napkin with optimized anionic strip.

Sanitary napkins and tampons, baby diapers and male and female incontinence pads and panty liners are known, which include a plurality of layers including an anionic strip releasing silver ions and ions of other negatively charged minerals, and a layer made according to the nano-silver nano technology.

EP-0251783B1 describes antimicrobial compositions which provide for the release of silver, gold and copper metal ions on biological fluids.

US-2595290, US-2066271 and EP-190504 describe antimicrobial compositions comprising silver as an antimicrobial agent.

EP 1 006 825 A1 describes an anti-microbial body care product such as a sanitary napkin comprising in the part contacting human or animal skin and/ or mucosa an organic matrix containing homogeneously dispersed particles of metallic silver having a particle size in the range of 1 to 50 nm. It is known that the more a membrane is waterproof, the more it retains heat and moisture, in addition to absorbing the biological fluid.

Disadvantageously, waterproofing is in contrast with the breathable capacity of the sanitary napkin.

Known sanitary napkins have a sizing problem: a good sanitary napkin (which absorbs more) and with said waterproofing and breathability features balanced with each other, is bulky and not very wearable. A greater comfort of use, on the other hand, inhibits the ability to provide the product with the anionic strip.

The object of the present invention is to implement a sanitary napkin for external use in which there occurs the trigger of the anionic reaction that causes the bactericidal function, keeping the ability to retain heat and moisture, the breathable capacity and the absorption capacity of the biological fluid balanced.

A further object of the present invention is to implement a sanitary napkin not bulky and easily wearable.

According to the invention, these objects are achieved by a sanitary napkin for external use comprising a total surface which is provided with a useful surface, characterized in that it comprises a nano silver treated layer, an anionic strip and an absorbent polymeric band,
said sanitary napkin having a length between 140 mm and 380 mm,
said anionic strip having a stable concentration of negative ions per cubic centimeters between 4100 and 8000, over an area between 3% and 34% of the total surface, and between 7% and 34% of the useful surface.

These and other features of the present invention will appear more clearly from the following detailed description of embodiments thereof, made by way of a non-limiting example with reference to the accompanying drawings, in which:

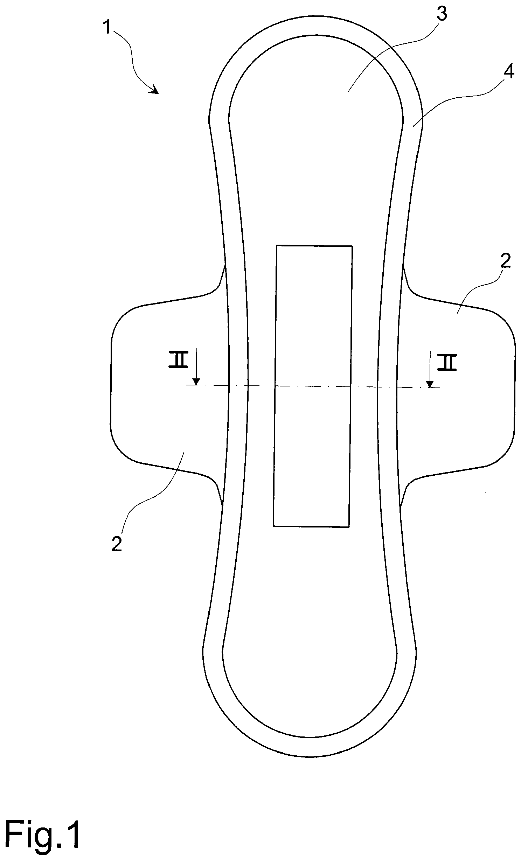

figure 1 shows a top plan view of a sanitary napkin according to the present invention;
figure 2 shows a sectional view according to line II-II in figure 1;
figure 3 shows a sectional view similar to that in figure 2 of a different embodiment of the sanitary napkin according to the present invention.

An external sanitary napkin 1, having a length between 170 mm and 380 mm, comprises two wings 2 connected to a useful surface 3 by means of an edge 4. The total surface 10 of the sanitary napkin 1 is defined by the sum of the useful surface 3 and of edge 4.

It is noted that by useful surface 3 it is meant the surface adapted to absorb the biological fluid.

The sanitary napkin 1 consists of a plurality of superimposed layers comprising:
- a perforated non-woven layer 5 with superficial nano silver treatment;
- a layer 6 of cotton mixed with other fibers, with a percentage of cotton between 50% and 80% by weight; alternatively, a single layer 8 in cotton 100% by weight with superficial nano silver treatment may be provided in place of the combination of layers 5 and 6;
- an anionic strip 7 (negative silver ions and other compositions) inserted between said layers 5, 6, having a width between 1 cm and 4 cm and a length between 4 cm and 11 cm; alternatively, the anionic strip 7 may be placed in contact above or below a single first layer 5 or 6 or 8 of the sanitary napkin 1, thus in a visible (if above) or not visible (if below) manner;
- a compact polymeric band 9 (SAP, super absorbent polymer), in contact with the cotton layer 6 or 8 having a thickness between 0.1 mm and 2 mm across the entire useful surface 4 of the sanitary napkin 1, or a plurality of small grains arranged loose across the entire useful surface 3 of the sanitary napkin 1; SAP 9 therefore is an absorbent, or rather very absorbent polymeric band;
- a perforated and breathable closing lower film 20.

A layer with features similar to the cotton layer 6 or with different features, i.e. with cellulose material to keep the external part cool, may be inserted between SAP 9 and the perforated layer 5.

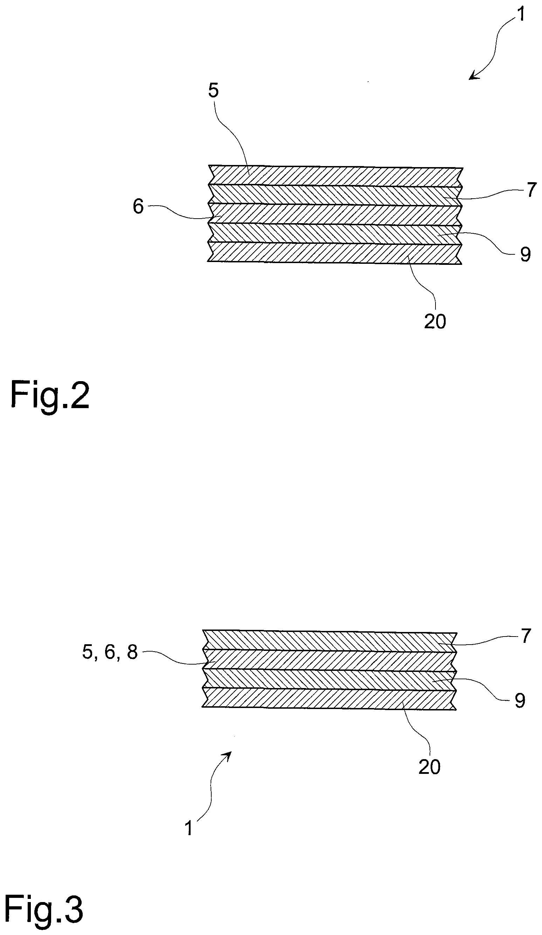

It is therefore understood that the arrangement of the layers in figure 2 is an example and may vary according to the above: for example, the polymeric band 9 may be integral with a cotton layer 5, 6 or 8.

Figure 3 shows an embodiment in which the anionic band 7 is over a single first layer 5 or 6 or 8 of the sanitary napkin 1.

The anionic strip 7 has a stable concentration of negative ions per cubic cm variable according to the size of the sanitary napkin 1.

For sanitary napkins 1 with total area surface 10 having a length between 170 mm and 225 mm, with useful surface 3 having a length between 150 mm and 210 mm, the anionic strip 7 has a stable concentration of negative ions per cubic cm between 4500 and 8000, over an area between 7% and 20% of the total surface 10, and between 14% and 34% of the useful surface 3.

For sanitary napkins 1 with total area surface 10 having a total length between 225 mm and 280 mm, with useful surface 3 having a length between 210 mm and 265 mm, the anionic strip 7 has a stable concentration of negative ions per cubic cm between 4300 and 8000, over an area between 5% and 14% of the total surface 10, and between 9% and 25% of the useful surface 3.

For sanitary napkins 1 with total area surface 10 having a total length between 280 mm and 380mm, with useful surface 3 having a length between 266 mm and 366 mm, the anionic strip 7 has a stable concentration of negative ions per cubic cm between 4100 and 8000, over an area between 3% and 10% of the total surface 10, and between 7% and 17% of the useful surface 3.

In anatomical panty liners and sports thong panty liners, having a length between 140 mm and 180 mm, the concentration of negative ions is between 4500 ions per cubic cm and 8000 ions per cubic cm.

Said gradation is essential not only for the bacteriological function but also for the compliance with the natural PH of the expression atmosphere, so that the polymers absorb and retain in less than 15 sec. a quantity of fluids with a specific weight equal to 30 times their weight.

The quantity of polymers in the polymeric band SAP 9 must be such as not to form, in the products made, a layer of thickness greater than 50% of the finished sanitary napkin, in order not to inhibit the efficacy of the anionic strip 7.

Types and quantities of the materials used in the prototypes may not be altered if not against a proven efficacy and balancing thereof.

The preferred quantity of ions is related to the quantity of polymer used: in fact, it retains the right amount of moisture such as to allow the efficacy of an anionic strip 7 of such dimensions as to grant the correct laying of the filtering membranes without them loosening or imparting a central and/or longitudinal bending to the sanitary napkin 1 not suitable to the correct use of the product, besides the bactericidal function thereof.

Application examples come from laboratory tests at Chelab srl in Resana, in the province of Treviso, which showed that out of the ranges mentioned above, the following occurs:
- an evident decrease in the absorption rate (0.04 ml/s versus 0.22 ml/s of the sanitary napkin according to the invention for 20 ml of liquid poured, with equal absorption capacity per g of the product, i.e. about 17.6 g);
- a net decrease in the radial diffusion of the liquid as the number of loadings increases (6.3 cm versus 8.1 cm of the sanitary napkin according to the invention at the 5th loading for a total of 25 ml poured);
- a high rewetting value, or the amount of liquid released by the sanitary napkin under pressure which is directly connected to the comfort of the product; the greater the feeling of dry, the lower the rewetting value (0.48 g versus 0.98 g of the sanitary napkin according to the invention on the 1 st loading for a total of 10 ml poured, 1.89 g versus 2.73 g of the sanitary napkin according to the invention on the 2nd loading for a total of 15 ml poured, 4.31 g versus 5.07 g of the sanitary napkin according to the invention on the 3rd loading for a total of 20 ml poured);
- a large amount and percentage of unabsorbed liquid as found in the "lateral flow" test (14.3 g versus 9.3 g of the sanitary napkin according to the invention, 72% versus 47% of the sanitary napkin according to the invention);
- a low breathability outwards.

An overall assessment results from the tests performed which achieves the objects of the present invention.

## Claims

1. A sanitary napkin (1) for external use comprising a total surface (10) which is provided with a useful surface (3) adapted to absorb the biological fluid, **characterized in that** it comprises a nano silver treated layer (5, 8), an anionic strip (7) and an absorbent polymeric band (9),
said sanitary napkin (1) having a length between 140 mm and 380 mm,
said anionic strip (7) having a stable concentration of negative ions per cubic centimeters between 4100 and 8000, over an area between 3% and 34% of the total surface (10), and between 7% and 34% of the useful surface (3).

2. A sanitary napkin (1) according to claim 1, **characterized in that** it comprises a perforated non-woven layer (5) with superficial nano silver treatment, and a layer (6) of cotton mixed with other fibers, with a percentage of cotton between 50% and 80% by weight.

3. A sanitary napkin (1) according to claim 1, **characterized in that** it comprises a single first layer (8) of cotton 100% by weight with superficial nano silver treatment.

4. A sanitary napkin (1) according to claims 2 or 3, **characterized in that** said absorbent polymeric band (9) is placed compact or in granules in contact with said layers (6, 8) with a thickness between 0.1 mm and 2 mm, on the whole useful surface (3) of the sanitary napkin (1).

5. A sanitary napkin (1) according to claims 2 or 4, **characterized in that** a layer of cotton or cellulose material may be inserted between the absorben polymeric band (9) and the perforated layer (5).

6. A sanitary napkin (1) according to any one of the preceding claims, **characterized in that** for sanitary napkins (1) having a length between 170 mm and 225 mm, with useful surface (3) having a length between 150 mm and 210 mm, the anionic strip (7) has a stable concentration of negative ions per cubic centimeters between 4500 and 8000, over an area between 7% and 20% of the total surface (10), and between 14% and 34% of the useful surface (3).

7. A sanitary napkin (1) according to any one of claims 1-5, **characterized in that** for sanitary napkins (1) having a length between 225 mm and 280 mm, with useful surface (3) having a length between 210 mm and 265 mm, the anionic strip (7) has a stable concentration of negative ions per cubic centimeters between 4300 and 8000, over an area between 5% and 14% of the total surface (10), and between 9% and 25 % of the useful surface (3).

8. A sanitary napkin (1) according to any one of claims 1-5, **characterized in that** for sanitary napkins (1) having a length between 280 mm and 380mm, with useful surface (3) having a length between 265 mm and 366 mm, the anionic strip (7) has a stable concentration of negative ions per cubic centimeters between 4100 and 8000, over an area between 3% and 10% of the total surface (10), and between 7% and 17 % of the useful surface (3).

## Patentansprüche

1. Hygienebinde (1) zur äußerlichen Verwendung, mit einer Gesamtfläche (10), die mit einer zum Absorbieren des biologischen Fluids ausgebildeten Nutzfläche (3) versehen ist, **dadurch gekennzeichnet, dass** sie eine Nanosilber-behandelte Schicht (5, 8), einen anionischen Streifen (7) und ein absorbierendes Polymerband (9) aufweist,
wobei die Hygienebinde (1) eine Länge zwischen 140 mm und 380 mm besitzt,
wobei der anionische Streifen (7) eine stabile Konzentration von negativen Ionen pro Kubikzentimeter zwischen 4100 und 8000, über einen Bereich zwischen 3% und 34% der Gesamtfläche (10) und zwischen 7% und 34% der Nutzfläche (3), aufweist.

2. Hygienebinde (1) nach Anspruch 1, **dadurch gekennzeichnet, dass** sie eine perforierte Vliesschicht (5) mit oberflächlicher Nanosilberbehandlung und eine Schicht (6) aus Baumwolle gemischt mit anderen Fasern, mit einem Anteil an Baumwolle zwischen 50 Gew.-% und 80 Gew.-%, aufweist.

3. Hygienebinde (1) nach Anspruch 1, **dadurch gekennzeichnet, dass** sie eine einfache erste Schicht (8) aus 100 Gew.-%-Baumwolle mit oberflächlicher Nanosilberbehandlung aufweist.

4. Hygienebinde (1) nach Anspruch 2 oder 3, **dadurch gekennzeichnet, dass** das absorbierende Polymerband (9) kompakt oder in Körnern in Kontakt mit den genannten Schichten (6, 8) mit einer Dicke zwischen 0,1 mm und 2 mm auf der ganzen Nutzfläche (3) der Hygienebinde (1) angeordnet ist.

5. Hygienebinde (1) nach Anspruch 2 oder 4, **dadurch gekennzeichnet, dass** zwischen dem absorbierenden Polymerband (9) und der perforierten Schicht (5) eine Schicht aus Baumwoll- oder Cellulosematerial eingefügt sein kann.

6. Hygienebinde (1) nach einem der vorangehenden Ansprüche, **dadurch gekennzeichnet, dass** für Hygienebinden (1) mit einer Länge zwischen 170 mm und 225 mm, mit Nutzfläche (3) mit einer Länge zwischen 150 mm und 210 mm, der anionische Streifen (7) eine stabile Konzentration von negativen Ionen pro Kubikzentimeter zwischen 4500 und 8000, über einen Bereich zwischen 7% und 20% der Gesamtfläche (10) und zwischen 14% und 34% der Nutzfläche (3), aufweist.

7. Hygienebinde (1) nach einem der Ansprüche 1 bis 5, **dadurch gekennzeichnet, dass** für Hygienebinden (1) mit einer Länge zwischen 225 mm und 280 mm, mit Nutzfläche (3) mit einer Länge zwischen 210 mm und 265 mm, der anionische Streifen (7) eine stabile Konzentration von negativen Ionen pro Kubikzentimeter zwischen 4300 und 8000, über einen Bereich zwischen 5% und 14% der Gesamtfläche (10) und zwischen 9% und 25% der Nutzfläche (3), aufweist.

8. Hygienebinde (1) nach einem der Ansprüche 1 bis 5, **dadurch gekennzeichnet, dass** für Hygienebinden (1) mit einer Länge zwischen 280 mm und 380 mm, mit Nutzfläche (3) mit einer Länge zwischen 265 mm und 366 mm, der anionische Streifen (7) eine stabile Konzentration von negativen Ionen pro Kubikzentimeter zwischen 4100 und 8000, über einen Bereich zwischen 3% und 10% der Gesamtfläche (10) und zwischen 7% und 17% der Nutzfläche (3), aufweist.

## Revendications

1. Serviette hygiénique (1) pour une utilisation externe comprenant une surface totale (10) qui est pourvue d'une surface utile (3) adaptée pour absorber le fluide biologique, **caractérisé en ce qu'**elle comprend une couche traitée en nano-argent (5, 8), une bande anionique (7) et une bande polymérique absorbante (9),
ladite serviette hygiénique (1) ayant une longueur comprise entre 140 mm et 380 mm,
ladite bande anionique (7) ayant une concentration stable d'ions négatifs par centimètres cubes entre 4100 et 8000, sur une surface comprise entre 3% et 34% de la surface totale (10), et entre 7% et 34% de la surface utile (3).

2. Serviette hygiénique (1) selon la revendication 1, **caractérisée en ce qu'**elle comprend une couche non tissée perforée (5) avec un traitement superficiel en nano-argent, et une couche (6) de coton mélangé à d'autres fibres, avec un pourcentage de coton entre 50% et 80% en poids.

3. Serviette hygiénique (1) selon la revendication 1, **caractérisée en ce qu'**elle comprend une seule première couche (8) de coton 100% en poids avec un traitement superficiel en nano-argent.

4. Serviette hygiénique (1) selon la revendication 2 ou la revendication 3, **caractérisée en ce que** ladite bande polymérique absorbante (9) est placée compacte ou en granulés en contact avec lesdites couches (6, 8) avec une épaisseur comprise entre 0,1 mm et 2 mm, sur toute la surface utile (3) de la serviette hygiénique (1).

5. Serviette hygiénique (1) selon la revendication 2 ou la revendication 4, **caractérisée en ce qu'**une couche de coton ou de matière cellulosique peut être insérée entre la bande polymérique absorbante (9) et la couche perforée (5).

6. Serviette hygiénique (1) selon l'une quelconque des revendications précédentes, **caractérisée en ce que**, pour des serviettes hygiéniques (1) ayant une longueur comprise entre 170 mm et 225 mm, avec une surface utile (3) ayant une longueur comprise entre 150 mm et 210 mm, la bande anionique (7) a une concentration stable d'ions négatifs par centimètres cubes entre 4500 et 8000, sur une surface comprise entre 7% et 20% de la surface totale (10) et entre 14% et 34% de la surface utile (3).

7. Serviette hygiénique (1) selon l'une quelconque des revendications 1 à 5, **caractérisée en ce que**, pour des serviettes hygiéniques (1) ayant une longueur comprise entre 225 mm et 280 mm, avec une surface utile (3) ayant une longueur comprise entre 210 mm et 265 mm, la bande anionique (7) a une concentration stable d'ions négatifs par centimètres cubes entre 4300 et 8000, sur une surface comprise entre 5% et 14% de la surface totale (10), et entre 9% et 25% de la surface utile (3).

8. Serviette hygiénique (1) selon l'une quelconque des revendications 1 à 5, **caractérisée en ce que**, pour des serviettes hygiéniques (1) ayant une longueur comprise entre 280 mm et 380 mm, avec une surface utile (3) ayant une longueur comprise entre 265 mm et 366 mm, la bande anionique (7) a une concentration stable d'ions négatifs par centimètres cubes entre 4100 et 8000, sur une surface comprise entre 3% et 10% de la surface totale (10) et entre 7% et 17% de la surface utile (3).
